# EUROPEAN PATENT APPLICATION

(11) **EP 3 346 450 A1**
(43) Date of publication of application: **11.07.2018**
(21) Application number: 17195490.2
(22) Date of filing: 09.10.2017
(51) Int. Cl.: G06T 19/20, A61B 5/055

(54) **METHOD FOR VISUALISING A MEDICAL IMAGE DATA SET, SYSTEM FOR VISUALISING A MEDICAL IMAGE DATA SET, COMPUTER PROGRAM PRODUCT AND COMPUTER-READABLE MEDIUM**

(71) Applicant: Siemens Healthcare GmbH, 91052 Erlangen (DE)
(72) Inventor: Haider, Sultan, 91052 Erlangen (DE); Huwer, Stefan, 91056 Erlangen (DE); Nittka, Mathias, 91083 Baiersdorf (DE); Zhao, Zhigen, 80805 München (DE); Alden, Joshua Shiloh, Atlanta, 30332 (US); Fusaro Guimaraes, Beatriz, 30180-091 Belo Horizonte, MG Brazil (BR)

(57) **Abstract**

The present invention suggests a method for visualising a medical image data set (11), in particular a magnetic resonance image data set, comprising:
- providing (101) the medical imaging data set (11),
- providing a first subset (21) of the medical image data set (11) related to a first parameter (51) and a second subset (22) of the medical image data set (11) related to a second parameter (52),
- assigning (103) a first colour to the first subset (21) and a second colour to the second subset (22),
- transferring (104) the first subset (21) in a first colour presentation (31) using the first colour and the second subset (32) in a second colour presentation (32) using the second colour and
- combining (105), in particular merging, the first colour presentation (31) and the second colour presentation (32) for visualising the medical imaging data set.

## Description

The present invention describes a method for visualising a medical image data set, a system for visualising a medical image data set, a computer program product and a computer-readable medium.

Visualising medical image data sets is well known in the state of the art. For example, a medical image data set is recorded by magnetic resonance imaging and the recorded medical imaging data set is visualized by reconstructing. As a result of such a reconstruction, a grey-scale imaging is presented on a display or a screen, wherein tissue types might be differentiated based on a grey-scale value assigned to each pixel of the visualized medical imaging data set. However, the unambiguous identification of the tissue might be hindered due the fact that different tissues are mapped to the same grey scale value. For identifying the specific tissue it is known to present different visualisations such as a first visualisation related to a Tl-relaxation time and as a second visualisation related to a T2-relaxation time. Thus, it is advantageously possible to present separately information being encoded in the first visualisation and the second visualisation respectively. However, by doing so additional information containing a structure of the data might get lost. Furthermore, an user has to switch his focus between the two visualisations. This is quite exhausting for the user and might lead to errors in analysing the visualized medical imaging data sets.

Alternatively, scatter plots might be presented wherein the plots show correlations between the Tl-relaxation time and the T2-relaxation time inside a selected region. However, this visualisation is limited to two parameters, namely the Tl-relaxation time and the T2-relaxation time. Furthermore, a separation of data points with respect to its spatial locations within the image is not visually clear, when a region or interest is too large.

Based on this background it is an object of the present invention to provide a method for visualising a medical imaging, wherein the visualized imaging data set supports easily the identification of tissue types.

This object is achieved by the method for visualising a medical imaging according to claim 1, by the system according to claim 13, the computer program product according to claim 14 and by the computer readable computer medium according to claim 15.

According to a first aspect of the present invention a method for visualising a medical image data set, in particular a magnetic resonance image data set, is provided, comprising:
- providing the medical imaging data set,
- providing a first subset of the medical image data set related to a first parameter and a second subset of the medical image data set related to a second parameter,
- assigning a first colour to the first subset and a second colour to the second subset,
- transferring the first subset in a first colour presentation using the first colour and the second subset in a second colour presentation using the second colour and
- combining, in particular merging, the first colour presentation and the second colour presentation for visualising the medical imaging data set.

In contrast to the state of the art all information being encoded in the first subset and the second subset are presented in advance in one common visualisation of the medical imaging data set. As a consequence, there is no need for the user to switch focus between a first visualisation and a second visualisation. Instead, the user is supported by the combination of the first colour presentation and the second colour presentation, for instance for identifying a specific type of tissue. Another advantage is that the method for visualising is not limited to two parameters, i. e. for example at least three subsets of the medical imaging data set are generated or provided and subsequently transferred to the respective colour presentation. As a consequence, more information can be included into one common visualisation. Furthermore, the method is not limited to a first colour presentation and a second colour presentation.

The term "combining the first colour presentation and the second colour presentation" preferably means that the first colour presentation and the second colour presentation are combined or merged pixel per pixel or voxel per voxel, i. e. a pixel of the first colour visualisation is combined with a corresponding pixel of the second visualisation or a voxel of the first colour visualisation is combined with a corresponding voxel of the second visualisation. For example, the pixels of the first and the second colour presentation are arranged in a two dimensional array and the pixels having the same location in each array are combined. Preferably by combining the first colour presentation and the second colour presentation, a third colour being different from the first colour and the second colour is generated and visualized.

The term "colour presentation" preferably means that an intensity value of a specific colour is assigned to each pixel or voxel. As a consequence, a high intensity is assigned to a tissue type and a low intensity is assigned to another tissue type. Preferably, the medical imaging data set is recorded by a magnetic resonance imaging device. In general, the first parameter and the second parameter represents such parameters such as a Tl-relaxtation time, a T2-relaxation time and/or a proton density. It is also conceivable that the first parameter and/or the second parameter are related to other tissue properties that are measured with magnetic resonance (MR)-methods, such as for example diffusion, perfusion and/or the like.

In particular, a control unit is provided, wherein the control unit comprises a processor being configured for executing at least one of the steps
- providing a first subset of the medical image data set related to a first parameter and a second subset of the medical image data set related to a second parameter,
- assigning a first colour to the first subset and a second colour to the second subset,
- transferring the first subset in a first colour presentation using the first colour and the second subset in a second colour presentation using the second colour and/or
- combining, in particular merging, the first colour presentation and the second colour presentation for visualising the medical imaging data set. The control unit might be incorporated into a workstation or a medical imaging device or might be part of a server, part of a system of server and/or a cloud. A workstation might be a (personal) computer, a virtual running machine on host hardware, a microcontroller, or an integrated circuit. As an alternative, the workstation can be a real or a virtual group of computers. Preferably, the workstation comprises a calculation unit and a memory unit. A calculation unit can comprise hardware elements and software elements, for example a microprocessor or a field programmable gate array. A memory unit can be embodied as non-permanent main memory (e. g. random access memory) or a permanent mass storage (e. g. a hard disk, USB stick, SD card, solid state disk). Preferably, the workstation can be part of the medical imaging device. It is also thinkable that at least one of the steps of the method is performed on a server or at the cloud.

Furthermore, it is provided that the common visualisation being result of combining the first colour presentation and the second colour presentation is transferred to a display or screen, such as a display of the workstation, a tablet, a smartphone or the like, and is monitored on the display of screen.

Particularly advantageous embodiments and features of the invention are given by the dependent claims as revealed in the following description. Features of different claim categories may be combined as appropriate to give further embodiments not described herein.

According to a preferred embodiment of the present invention it is provided that the first colour presentation and the second colour presentation are realized in form of layers being overlapped for combining. As a consequence, the different presentations can be combined pixel by pixel in an easy way. Furthermore, it is possible to selectively remove the first colour presentation and/or the second colour presentation by removing the respective layer in the visualisation. Be reducing the number of layer the operator can switch, in particular individually switch, to a presentation that contains less information, when too many information are agglomerated in the common visualisation.

In another preferred embodiment of the present invention, it is provided that a colour map is used for choosing the first colour and/or the second colour. In particular, it is provided that the colour map is a RGB-map comprising the basic colours red, green and blue or a CMYK-map comprising the basic colours cyan, magenta, yellow and black. In particular, it is provided that the first colour and the second colour are mixed such that a different colour result is generated by mixing. Since RGB is the summation of light values, the colours generated by the representation are comparatively bright and the contrast is sharp. Choosing a CMYK presentation lead to a presentation, wherein grey matter shows up as green colour and a tumor shows up as orange mass for example.

Preferably, for transferring the first subset into the first colour presentation a grey scale value is transferred to a colour scale value of the first colour for each pixel of the first subset and/or wherein for transferring the second subset into the second colour presentation a grey scale value is transferred to a colour scale value of the second colour for each pixel of the second subset. In particular, the colour scale value represents an intensity of the colour. Thus by mixing the first colour presentation and the second presentation, the assigned intensities determine the colour being result of mixing the first colour presentation and the second colour presentation. As a result, different tissues can be identified by different colours, because each tissue has a specific intrinsic value for the first parameter and/or second parameter. This allows a precise identification of the type of tissue.

In particular, it is provided that the first subset and/or the second subset are result of correlating a subset related to a third parameter, for example the T1-relaxtation time, and a subset related to a fourth parameter, for example the T2-relaxtation time. That means the first subset related to the first parameter and the second sunset related to the second parameter are preferably not directly extracted from the medical imaging data set, but are result of a data manipulation or correlation between subsets or between visualisations being available from the medical imaging data set. Thus, it is advantageously possible to identify correlations between the subset related to the third parameter and the subset related to the fourth parameter quantitatively for supporting a further classification of tissue types. For example, the correlation of the Tl-relaxation time and the T2-relaxation time can be used for identifying the tissue type. In particular, the subset related to the third parameter and the subset related to the fourth parameter are extracted from the medical imaging data set and the correlation is performed pixel per pixel. It is also thinkable that the first subset is result of a correlation and the second subset is directly related to the Tl-relaxation time, the T2-relaxation time or the like. For assigning intensity values to the first subset and/or the second subset it is preferably provided that the method comprises:
- identifying a cluster by correlating the third parameter and the fourth parameter in a correlation level;
- determining a centre of the identified cluster;
- determining a distance between each point in the cluster to the centre of the identified cluster; and
- assigning an intensity to the pixel in the first subset and/or the second subset based on the determined distance. Thus, it is advantageously possible to assign the result of the correlation to an intensity value that can be used for combining the first colour presentation and the second colour presentation. The term "cluster" preferably describes an agglomeration of similar correlation values located in the same region of the first subset and/or second subset. Furthermore, each cluster centroid/centre represent a reference point. Depending on the chosen clustering model, cluster centroids or centers can be defined as the means of all data points belonging to such a cluster, or as the centre of a probability density function of a distribution model, for example a Gaussian distribution for a Gaussian mixture model. For each reference point (defined by the cluster centroid) the distance between the reference point and all data point within a region of interest can then be calculated. Subsequently, the calculated distance is mapped back to the position of the corresponding pixel and thus generating the first colour presentation of distance based image data.

Preferably, the distance is determined by a Euclidean distance, by a Mahalanobis distance, a cosine simailarity and Manhatten distance and/or the like. The advantage of using a Euclidean distance is providing a stable performance across different quality of data sets and different clustering methods, since the calculation of Euclidean distance does not require any additional information such as a covariance matrix. The advantage of using a Mahalanobis distance is taking into account the shape and size of the cluster and therefore sharper images can be produced compared to a visualisation being result of calculating the Euclidean distance. Preferably, it is provided that the covariance matrix is taken into account.

In another embodiment of the present information, it is provided that a principle component analysis is used for reducing the number of parameters. Thus, it is possible to reduce the number of colour presentation to the maximal number of basic colour being available in the selected colour map (maximal number of colour channels in the RGB representation: 3; maximal number of colour channels in the CMYK representation: 4). As a consequence, a random number of subsets each being correlated to a parameter can be combined within a common visualisation.

Preferably, it is provided that a machine learning mechanism is used for identifying the cluster. For example, a deep learning mechanism is used to train an artificial network being able to identify a cluster within a correlation between the third parameter and the fourth parameter. Advantageously the machine learning mechanism establishes a correlation between the third parameter and the fourth parameter that can be used for identifying a specific type of tissue. In particular, the artificial network is trained by analysed medical imaging data set from the past and/or by artificial medical imaging data sets generated for training.

Furthermore, it is provided that an organ and/or abnormality is identified by correlating a third subset related to a third parameter and/or a fourth subset related the fourth parameter. It is thinkable that by comparing the correlation of the third subset and the fourth subset to previous analysed medical imaging data sets, in particular to previous correlations, the organ and/or the abnormality recorded by the medical imaging device are automatically identified. Preferably, the user is informed by a report data set that contains the information about the organ and/or the abnormality.

In another preferred embodiment it is provided that the first subset and the second subset are extracted from the medical imagine data set, in particular the same medical imaging data set. By extracting the first subset and the second subset from the same medical image data sets undesired shifts between the first and the second subset and consequently between the first colour presentation and the second colour presentation can be avoided.

According to another preferred embodiment of the present invention, it is provided that the medical imaging data set is recorded in a single scan. Thus, it can be advantageously be avoided that the medical imaging data set for the first subset and another medical imaging data set are recorded subsequently. As a consequence, the method for visualizing is accelerated.

According to another aspect of the present invention, a system for visualising a medical image data set is provided, wherein the system is configured for:
- providing the medical imaging data set,
- providing a first subset of the medical image data set related to a first parameter and a second subset of the medical image data set related to a second parameter,
- assigning a first colour to the first subset and a second colour to the second subset,
- transferring the first subset in a first colour presentation using the first colour and the second subset in a second colour presentation using the second colour and
- combining, in particular merging, the first colour presentation and the second colour presentation for visualising the medical imaging data set. In particular, the system comprises a control unit having a processor being configured for performing at least one of the steps mentioned above.

Another aspect of the present invention is a computer program product for carrying out the steps of the method according to the present invention when the computer program product is loaded into a memory of a programmable device.

A further aspect of the present invention is a computer-readable medium on which is stored a program elements that can be read and executed by a computer unit in order to perform steps of the method according to the present invention when the program elements are executed by the computer unit. Preferably, the computer unit and/or the programmable device are incorporated into the system.

In the drawing:
Fig. 1 shows schematically a system for visualizing a medical imaging data set according to a first embodiment of the present invention
Fig. 2 shows a flow diagram illustrating a part of the method according to a second embodiment of the present invention
Fig. 3 shows a part of a system for visualizing a medical imaging data set according to a third embodiment of the present invention
Fig. 4 shows a flow diagram illustrating a part of the method according to a fourth embodiment of the present invention.
Fig. 5 shows a flow diagram illustrating a method according to a fifth embodiment of the present invention.

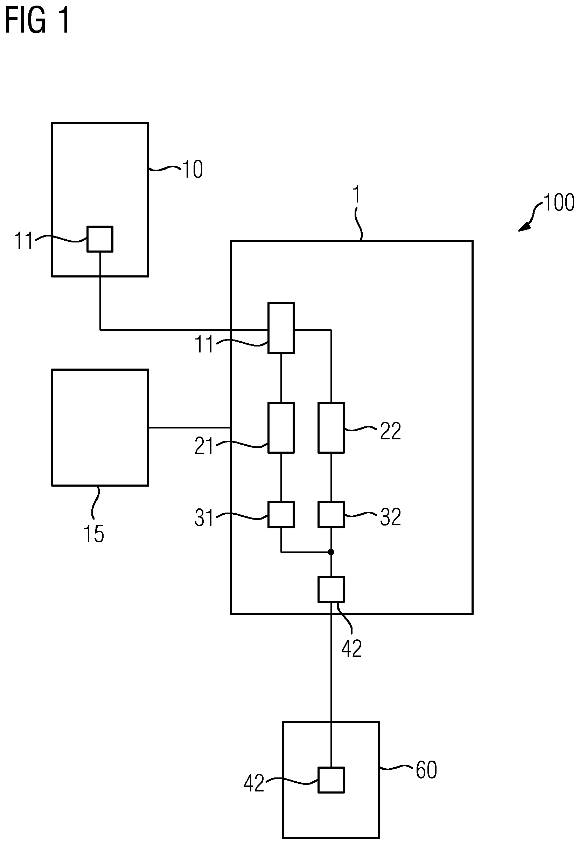

In figure 1 a system 100 for visualizing a medical imaging data 11 set is presented schematically. In general, the medical imaging data set 11 is provided, in particular recorded, by a medical imaging device 10, in particular a magnetic resonance imaging (MRI) device. According to the state of the art, the medical imaging data set 11 is usually presented in a grey-scale image, wherein different grey levels might indicate different tissue types. However, the grey levels cannot assigned unambiguously to one specific tissue type in a common visualization. In other words: in one visualisation a white region might represent a tumour or another tissue. For this reasons other visualisations of the medical imaging data set 11 are needed to verify the presence of the tumour. For example, a first subset 21 of the medical imaging data set 11 corresponding to a first parameter 51 and a second subset 22 of the medical imaging data set 11 corresponding to a second parameter 52 are visualized next to each other for comparing, for example on a screen 60 or a display according to the state of the art. Thereby, the first subset 21 preferably represents a visualisation based the first parameter 51 and the second subset represented a visualisation based on the second parameter 52. For the magnetic resonance imaging proceeding the first parameter 51 and/or the second parameter 52 might be a relaxation time T1, a relaxation time T2, a proton density and/or other parameters related to tissue properties, e.g. diffusion, perfusion, magnetic transfer, BOLD or the like.

Preferably, it is provided that the medical imaging data set 11 is recorded in a single scan and the first subset 21 and/or the second subset 22 are extracted from the medical imaging data set 11 being result of the single scan. By recording the medical imaging data set 11 in a single scan and subsequently extracting the first subset 21 and/or the second subset 21, it is advantageously possible to provide quantitative measurements in a time efficient way. Alternatively, it is thinkable that the first subset and the second subset are recorded separately by the medical imaging device.

In order to support identifying a critical abnormality such as a tumour, the system 100 presented in figure 1 is provided. Thereby the system 100 comprises a control unit 1 being configured for providing a first subset 21 of the medical imaging data set 11 and a second subset 22 of the medical imaging data set 11, in particular by extracting the first subset 21 and the second subset 22 from a medical imaging data set 11 recorded in a single scan. Further, a first colour is assigned to the first subset 21 and a second colour is assigned to the second subset 22, wherein the assignment is performed automatically and/or by entering via an input device 15. Preferably, the first colour and the second colour are basic colours of a colour model such as RGB map (three basic colours) or CMYK map (four basic colours). For example, the first colour and/or the second colour are red, green or blue in the case of using the RGB model.

Furthermore, it is provided that the first subset 21 is transferred to a first colour visualisation 31 and the second subset 22 is transferred to a second colour visualisation 32. Thereby, for transferring the first subset 21 in the first colour presentation 31 a grey scale value assigned to the first subset 21 is transferred to a colour scale value of the first colour for each pixel of the first subset 21, and/or wherein for transferring the second subset 22 in the second colour presentation a grey scale value assigned to the second subset 21 is transferred to a colour scale value of the second colour for each pixel of the second subset 21. For example for each pixel of the first subset 21 a value between 0 and 1 is assigned to labelling the grey scale. This value might be transferred to an intensity value of the first colour in the first colour visualisation 31.

Subsequently, the first colour visualisation 31 and the second colour visualisation 32 are combined, in particular merged. As a result, the medical imaging data set 11 is visualized such that the information included in the first subset 31 and the second subset 32 are respectively presented in one common visualisation 42. Preferably, the first colour and the second colour are mixed to a final colour, in particular at each pixel. Furthermore, it is provided that the first colour presentation and the second colour presentation are put on top of each other for combining or merging them.

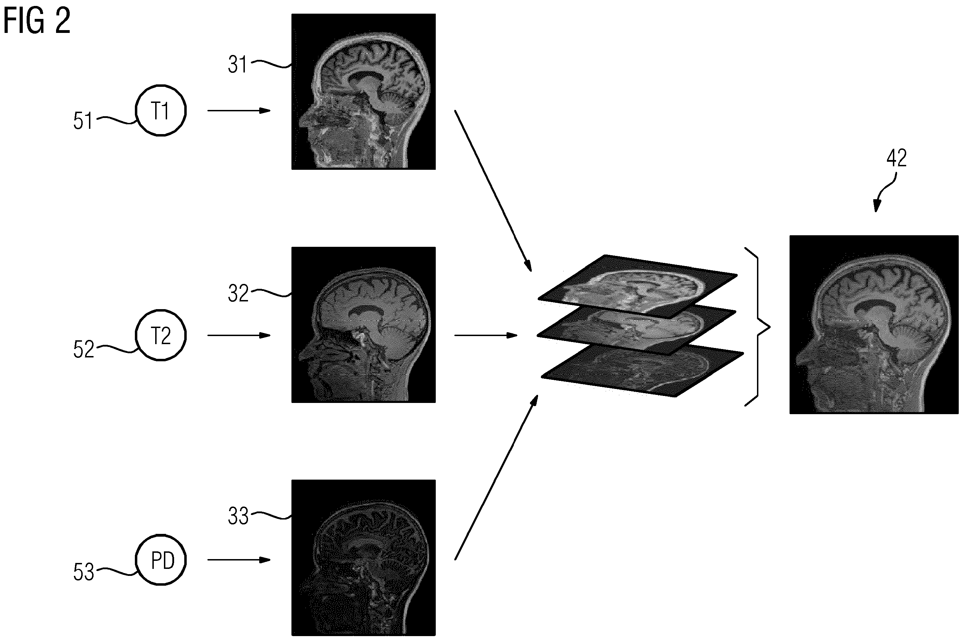

In figure 2 a flow diagram is presented that illustrates providing a first colour presentation 31, a second colour presentation 32 and a further second colour presentation 33 (for a further second parameter 53) and subsequently combining the first colour presentation 31, a second colour presentation 32 and a further second colour presentation 32 for generating a common visualisation 42.

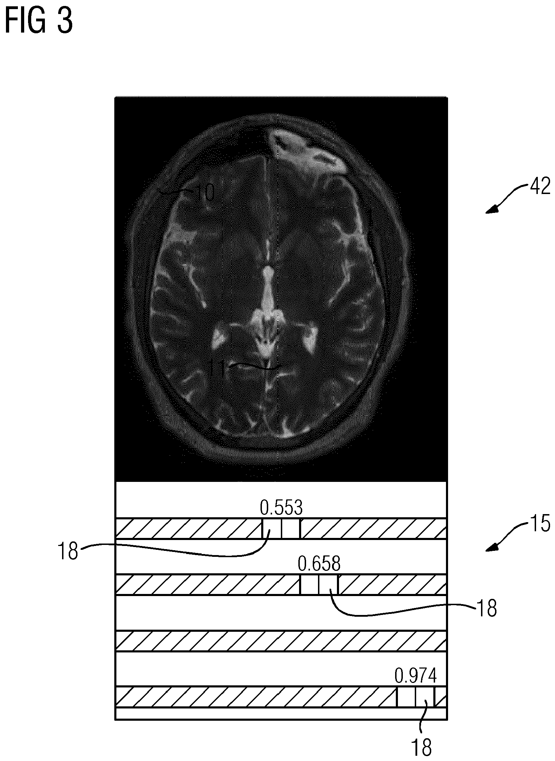

In figure 3 a part of a system 100 for visualizing a medical imaging data set 11 according to a third embodiment of the present invention is shown. In particular, according to the third embodiment, an input device 15 is provided, wherein the input device 15 is configured such that on the screen 60 a slider 18 for each parameter or colour is monitored next to the common visualisation 42. By shifting the respective slider 18 the influence of the first colour presentation 31 or the second colour presentation 32 in the common visualisation 42 of the medical imaging data set 11 is weighted respectively. Thus, the input device 15 is configured for weighting influence of the first colour presentation 31 or the second colour presentation 32 in the visualisation of the medical imaging data set 11. Preferably, this is done by adding an offset value and then wrapping the values back into a 0 to 1 range. In particular, the input device 15 comprises the slider 18 for adapting the offset value for the first colour presentation 31 and a further slider 18 for adapting the offset value for the second colour presentation. Thus, an operator can adjust the visualisation to his preference or for highlighting a specific and individualized outcome.

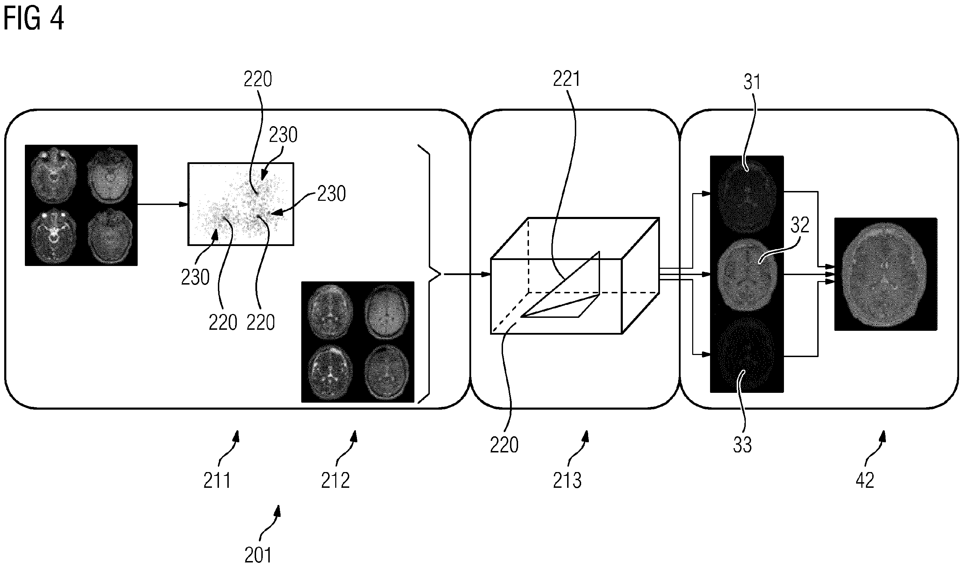

In figure 4 a flow diagram illustrating a part of the method according to a fourth embodiment of the present invention is illustrated. The method mainly corresponds to the method described in the context of the figures 1 and 2 and differs only in the kind of the first parameter 51 and the second parameter 52. In particular, the first parameter 51 in figure 4 is represented by a correlation of a third 54 parameter, for example the Tl-relaxation time, and a fourth parameter 55, for example a T2-relaxation time, and the second parameter 52 is represented by a second correlation of the third parameter 54 and the fourth parameter 55. In other words: instead of using a T1 related first subset and a T2 related second subset the result of a correlation of the Tl-relaxation time and the T2 relaxation time is used as first subset. Thereby, for example the Tl-relaxation time is plotted versus the T2-relaxation time for identifying 211 the correlation between the first subset 21 and the second subset 22 in a correlation level. As a result, a cluster 230, i. e. region with an increased correlation, can be identified in a correlation step. It is also thinkable that several cluster 230 are identified. These clusters 230 can be used to identify special tissue types for example. In order to incorporate the information being encoded in the correlation,
- determining 212 a centre 220 of each cluster and
- determining 213 a distance 221 to the centre 230 for each point in the cluster 230 in the correlation level is provided. As a consequence, it is possible to assign an intensity value to each determined distance 221, in particular in the first colour presentation 31 and/or the second colour presentation 32 of the medical image data set 11. Subsequently, the first colour presentation 51 and the second colour presentation 52 originating from the correlation of the Tl-relaxation time and the T2-relaxation time are combined to the common visualisation 42 of the medical imaging data set 11.

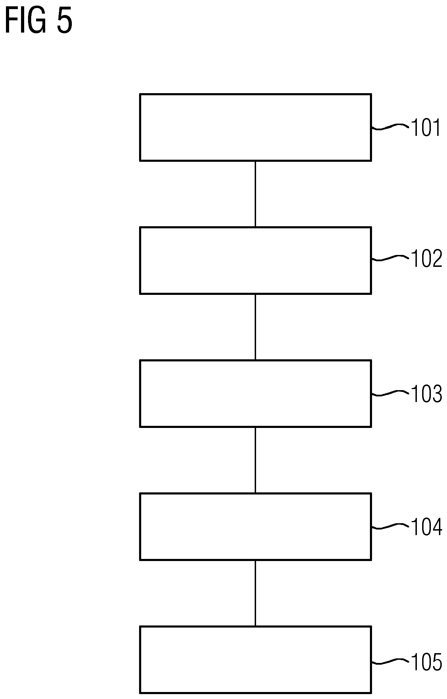

In figure 5 a flow diagram of a method according the present invention is illustrated, wherein the method comprises:
- providing 101 the medical imaging data set 11,
- providing, in particular extracting 102, a first subset 21 of the medical image data set 11 related to a first parameter 51 and a second subset 22 of the medical image data set 11 related to a second parameter 52,
- assigning 103 a first colour to the first subset 21 and a second colour to the second subset 22,
- transferring 104 the first subset 21 in a first colour presentation 31 using the first colour and the second subset 32 in a second colour presentation 32 using the second colour and/or
- combining 105, in particular merging, the first colour presentation 31 and the second colour presentation 32 for visualising the medical imaging data set 11.

## Claims

1. Method for visualising a medical image data set (11), in particular a magnetic resonance image data set, comprising:
- providing (101) the medical imaging data set (11),
- providing a first subset (21) of the medical image data set (11) related to a first parameter (51) and a second subset (22) of the medical image data set (11) related to a second parameter (52),
- assigning (103) a first colour to the first subset (21) and a second colour to the second subset (22),
- transferring (104) the first subset (21) in a first colour presentation (31) using the first colour and the second subset (32) in a second colour presentation (32) using the second colour and
- combining (105), in particular merging, the first colour presentation (31) and the second colour presentation (32) for visualising the medical imaging data set.

2. Method according to claim 1, wherein the first colour presentation (31) and the second colour presentation (32) are realized in form of layers being overlapped for combining (105) .

3. Method according to one of the preceding claims, wherein a colour map is used for choosing the first colour and/or the second colour.

4. Method according to one of the preceding claims, wherein for transferring the first subset (21) into the first colour presentation (31) a grey scale value is transferred to a colour scale value of the first colour for each pixel of the first subset (31), and/or wherein for transferring the second subset (22) into the second colour presentation (32) a grey scale value is transferred to a colour scale value of the second colour for each pixel of the second subset (22).

5. Method according to one of the preceding claims, wherein the first subset and/or the second subset are result of correlating (201) a subset related to a third parameter and a subset related to a fourth parameter.

6. Method according to claim 5, wherein for assigning intensity values to the first subset (21) and/or the second subset (22)
- identifying (211) cluster by correlating (201) the third parameter and the fourth parameter in a correlation level
- determining (212) a centre (220) of the identified cluster (230)
- determining (213) a distance (220) between each point in the cluster (230) to the centre (220) of the identified cluster (230) and
- assigning (214) an intensity to the pixel in the first subset and/or the second subset based on the determined distance (221) .

7. Method according to claim 5 or 6, wherein a principle component analysis is used for reducing the number of parameters.

8. Method according to one of the preceding claims, wherein a machine learning mechanism is used for identifying (211) the cluster (230).

9. Method according to one of the preceding claims, wherein an organ and/or abnormality is identified by correlating a subset related to a third parameter and a further subset related the fourth parameter.

10. Method according to one of the preceding claims, wherein the first subset and the second subset are extracted from a medical imagine data set.

11. Method according to one of the preceding claims, wherein the medical imaging data set is recorded in a single scan.

12. Method according to one of the preceding claims, wherein the first colour presentation and/or the second colour presentation are weighted, in particular via an input device (15) .

13. System (100) for configuring a medical instrument (10) comprising the medical instrument (10) and a server (100), wherein the system is configured for:
- providing (101) the medical imaging data set,
- providing a first subset (21) of the medical image data set (11) related to a first parameter (51) and a second subset (22) of the medical image data set (11) related to a second parameter (52),
- assigning (103) a first colour to the first subset (21) and a second colour to the second subset (22),
- transferring (104) the first subset (21) in a first colour presentation (31) using the first colour and the second subset (22) in a second colour presentation (32) using the second colour and
- combining (105), in particular merging, the first colour presentation (31) and the second colour presentation (32) for visualising the medical imaging data set (42).

14. Computer program product for carrying out the steps of the method according to any of claims 1 to 12 when the computer program product is loaded into a memory of a programmable device.

15. Computer-readable medium on which is stored a program elements that can be read and executed by a computer unit in order to perform steps of the method according to any of the claims 1 to 12 when the program elements are executed by the computer unit.

## Amended claims

### Amended claims in accordance with Rule 137(2) EPC.

1. Method for visualising a medical image data set (11), in particular a magnetic resonance image data set, comprising:
- providing (101) the medical imaging data set (11),
- providing a first subset (21) of the medical image data set (11) related to a first parameter (51) and a second subset (22) of the medical image data set (11) related to a second parameter (52),
- assigning (103) a first colour to the first subset (21) and a second colour to the second subset (22),
- transferring (104) the first subset (21) in a first colour presentation (31) using the first colour and the second subset (32) in a second colour presentation (32) using the second colour and
- combining (105), in particular merging, the first colour presentation (31) and the second colour presentation (32) for visualising the medical imaging data set,
wherein the first subset and/or the second subset are the result of correlating (201) a subset related to a third parameter and a subset related to a fourth parameter, wherein for assigning intensity values to the first subset (21) and/or the second subset (22), the following steps are carried out:
- identifying (211) a cluster by correlating (201) the third parameter and the fourth parameter in a correlation level,
- determining (212) a centre (220) of the identified cluster (230),
- determining (213) a distance (220) between each point in the cluster (230) to the centre (220) of the identified cluster (230), and
- assigning (214) an intensity to the pixel in the first subset and/or the second subset based on the determined distance (221).

2. Method according to claim 1, wherein the first colour presentation (31) and the second colour presentation (32) are realized in form of layers being overlapped for combining (105) .

3. Method according to one of the preceding claims, wherein a colour map is used for choosing the first colour and/or the second colour.

4. Method according to one of the preceding claims, wherein for transferring the first subset (21) into the first colour presentation (31) a grey scale value is transferred to a colour scale value of the first colour for each pixel of the first subset (31), and/or wherein for transferring the second subset (22) into the second colour presentation (32) a grey scale value is transferred to a colour scale value of the second colour for each pixel of the second subset (22).

5. Method according to one of the preceding claims, wherein a principle component analysis is used for reducing the number of parameters.

6. Method according to one of the preceding claims, wherein a machine learning mechanism is used for identifying (211) the cluster (230).

7. Method according to one of the preceding claims, wherein an organ and/or abnormality is identified by correlating a subset related to a third parameter and a further subset related the fourth parameter.

8. Method according to one of the preceding claims, wherein the first subset and the second subset are extracted from a medical imagine data set.

9. Method according to one of the preceding claims, wherein the medical imaging data set is recorded in a single scan.

10. Method according to one of the preceding claims, wherein the first colour presentation and/or the second colour presentation are weighted, in particular via an input device (15).

11. System (100) for configuring a medical instrument (10) comprising the medical instrument (10) and a server (100), wherein the system is configured for:
- providing (101) the medical imaging data set,
- providing a first subset (21) of the medical image data set (11) related to a first parameter (51) and a second subset (22) of the medical image data set (11) related to a second parameter (52),
- assigning (103) a first colour to the first subset (21) and a second colour to the second subset (22),
- transferring (104) the first subset (21) in a first colour presentation (31) using the first colour and the second subset (22) in a second colour presentation (32) using the second colour and
- combining (105), in particular merging, the first colour presentation (31) and the second colour presentation (32) for visualising the medical imaging data set (42) wherein the first subset and/or the second subset are result of correlating (201) a subset related to a third parameter and a subset related to a fourth parameter,
wherein for assigning intensity values to the first subset (21) and/or the second subset (22)
- identifying (211) cluster by correlating (201) the third parameter and the fourth parameter in a correlation level
- determining (212) a centre (220) of the identified cluster (230)
- determining (213) a distance (220) between each point in the cluster (230) to the centre (220) of the identified cluster (230) and
- assigning (214) an intensity to the pixel in the first subset and/or the second subset based on the determined distance (221).

12. Computer program product for carrying out the steps of the method according to any of claims 1 to 10 when the computer program product is loaded into a memory of a programmable device.

13. Computer-readable medium on which is stored a program elements that can be read and executed by a computer unit in order to perform steps of the method according to any of the claims 1 to 10 when the program elements are executed by the computer unit.
